Europäisches Patentamt

European Patent Office

Office européen des brevets

(19)

(11) Publication number: **0 128 510**
A2

(12) # EUROPEAN PATENT APPLICATION

(21) Application number: **84106422.3**

(22) Date of filing: **05.06.84**

(51) Int. Cl.³: **C 07 C 19/08, C 07 C 17/20**

(30) Priority: **07.06.83 JP 101309/83**
**11.06.83 JP 104626/83**
**11.06.83 JP 104627/83**

(43) Date of publication of application: **19.12.84**
**Bulletin 84/51**

(84) Designated Contracting States: **DE NL**

(71) Applicant: **SHOWA DENKO K.K., 13-9, Shiba Daimon 1-chome, Minato-ku, Tokyo 105 (JP)**
Applicant: **HITACHI, LTD., 6, Kanda Surugadai 4-chome Chiyoda-ku, Tokyo 100 (JP)**

(72) Inventor: **Takayama, Shigeru, No. 4-11, Akamatsu-cho, Chigasaki-shi Kanagawa (JP)**
Inventor: **Takaichi, Tsuyoshi, Takinosawa-danchi 4-101 Shonan-life-town, No. 1643, Endoh Fujisawa-shi Kanagawa (JP)**
Inventor: **Nakayama, Hidetoshi, No. 2, Shiomidai 3-chome, Isogo-ku Yokohama-shi Kanagawa (JP)**
Inventor: **Kawasaki, Hiroaki, No. 1993-57, Ohzenji Asao-ku, Kawasaki-shi Kanagawa (JP)**
Inventor: **Hashimoto, Norikazu, Nanyodai 50-14 No. 168-90, Shimoyuge, Hachiohji-shi Tokyo (JP)**
Inventor: **Kawamoto, Yoshifumi, No. 3511-14, Kawajiri Shiroyama-cho, Tsukui-gun Kanagawa (JP)**

(74) Representative: **Patentanwälte Henkel, Pfenning, Feiler, Hänzel & Meinig, Möhlstrasse 37, D-8000 München 80 (DE)**

(54) **Process for producing difluoromethane.**

(57) Difluoromethane is produced in high yield by reacting dichloromethane and hydrogen fluoride in a gaseous phase in the presence of at least one catalyst selected from (i) a catalyst consisting essentially of aluminum fluoride or a mixture of aluminum fluoride and (ii) a catalyst comprising from 0.05 to 1 part by weight of ferric chloride on 1 part by weight of activated carbon carrier.

EP 0 128 510 A2

# PROCESS FOR PRODUCING DIFLUOROMETHANE

## FIELD OF THE INVENTION

The present invention relates to a process for producing difluoromethane and, more particularly, to a process for producing difluoromethane in high yield by reacting dichloromethane with hydrogen fluoride in a gaseous phase in the presence of a catalyst.

## BACKGROUND OF THE INVENTION

Many processes for producing fluorocarbons which comprise reacting aliphatic halogenated hydrocarbons with hydrogen fluoride (HF) have hitherto been proposed and are described, e.g., in U.S. Patent 3,632,834, European Patent Application (OPI) Nos. 56,548 and 36,123 and British Patents 1,369,870 and 1,201,588, etc. However, halogenated hydrocarbons employed in the foregoing reaction are limited to those having at least 2 carbon atoms, carbon tetrachloride ($CCl_4$) or chloroform $CHCl_3$ of methane type compounds; furthermore, there is scarcely any literature on a process for producing difluoromethane ($CH_2F_2$) using dichloromethane ($CH_2Cl_2$) and HF.

In general, it is described in various reports that it is difficult to produce $CH_2F_2$ with high efficiency. It is described, for example, in Chemistry and

-2-

Industry of Fluorine Compounds, page 267, published December in 1977 by CMC Co., Ltd. that $-CCl_3$ group is highly reactive and substituted stepwise from $-CCl_2F$ to $-CClF_2$ but it is difficult to fluorinate it to reach $-CF_3$; further, the $-CHCl_2$ group has poor reactivity and although it can be substituted to reach $-CHClF$, it is difficult to further substitute it to reach $-CHF_2$; furthermore, as hydrogen in the molecule increases, the reactivity becomes poor, and decomposition and side reactions are liable to occur.

In fact, only a process involving a gaseous reaction of $CH_2Cl_2$ and HF using chromic trioxide as a catalyst (Japanese Patent Publication No. 3004/67) is disclosed as the process for producing $CH_2F_2$. However, the process yields only 43% $CH_2F_2$ based on the raw material $CH_2Cl_2$, and also results in serious loss of $CH_2Cl_2$, which is believed to be caused due to side reactions.

### SUMMARY OF THE INVENTION

An object of the present invention is to provide a process for producing difluoromethane in which side reactions are extremely minimized and the desired $CH_2F_2$ can be obtained in high yield.

As a result of wide range of and detailed investigations on the process comprising the reaction of $CH_2Cl_2$ and HF in a gaseous phase, the present inven-

- 2 -

tors have discovered that by performing the reaction under appropriate conditions using a specific catalyst, side reactions can be extremely minimized and $CH_2F_2$ can be obtained in high yield, and thus the present inventors have accomplished a process for producing $CH_2F_2$, that is extremely advantageous from an economical standpoint.

Namely, the present invention is directed to a process for producing difluoromethane which comprises reacting $CH_2Cl_2$ with HF in a gaseous phase in the presence of at least one catalyst selected from (i) a catalyst consisting essentially of aluminum fluoride or a mixture of aluminum fluoride and chromium fluoride and (ii) a catalyst obtained by depositing 0.05 to 1 part by weight of ferric chloride on 1 part by weight of activated carbon   carrier.

### DETAILED DESCRIPTION OF THE INVENTION

The catalyst (i) consisting essentially of aluminum fluoride or a mixture of aluminum fluoride and chromium fluoride may be carried on porous carriers such as activated carbon   etc., or may also be mixed with porous carriers followed by molding. For example, in the case of using aluminum fluoride ($AlF_3$) alone or aluminum fluoride and chromium trifluoride ($CrF_3$) in combination, they may be employed after molding as they are, as a mixture with porous carriers followed by molding or by carrying them on porous carriers.

In the case of using them after depositing on carriers, the proportion of aluminum fluoride or the mixture of aluminum fluoride and chromium fluoride is at least 1 wt%, and preferably from 2 to 20 wt% (calculated as Aℓ or Aℓ+Cr), based on the weight of the carriers. In the case of using them as a mixture with carriers, the proportion of aluminum fluoride or the mixture of aluminum fluoride and chromium fluoride is at least 1 wt%, and preferably from 2 to 30 wt% (calculated as Aℓ or Aℓ+Cr), based on the weight of the carriers.

The catalyst (i) can be obtained by subjecting an aluminum compound or a mixture of the aluminum compound and a chromium compound to fluorinating treatment. In this case, it is preferred that these compounds be subjected to fluorinating treatment after they are molded, after they are mixed with porous carriers followed by molding, or after they are deposited on carriers. Specific examples of chromium compounds include chromium oxide, chromium hydroxide, chromium chloride, chromium nitrate, etc., which are anhydrous or possess crystalline water; chromic acid salts such as ammonium chromate, etc. Specific examples of aluminum compounds include oxides (e.g. activated alumina), chlorides hydroxides, nitrates,

etc., of aluminum that are anhydrous or possess crystalline water. Of these chromium compounds and aluminum compounds, hydroxides, oxides and chlorides are preferably employed. $CrF_3$ or $AlF_3$ may also be partly incorporated into the aforesaid chromium compounds or aluminum compounds. In the case of using the chromium compounds and the aluminum compounds as the mixture, it is desired that the mixing ratio (by weight) of Al in the aluminum compound to Cr in the chromium compound be in the range of 0.1/1 to 50/1, and preferably be from 0.3/1 to 20/1. When the ratio of Al to Cr is overly large or small, side reactions are likely increased.

As the method for the fluorinating treatment described above, fluorocarbons such as $C_2Cl_3F_3$, $CClF_3$, etc., HF, or gases obtained by diluting the foregoing gases with inert gas such as $N_2$, etc., can be employed. In the case that the fluorinating treatment is carried out at relatively high temperatures, it is preferred to use diluted gases.

It is desired that the fluorinating treatment be performed at 150 to 500°C, and preferably at 200 to 450°C until the consumption of HF or fluorocarbons introduced is not substantially observed. The pressure is not particularly limited, but the treatment is generally conducted under normal atmospheric pressure.

- 6 -

The fluorinating treatment of the aforesaid aluminum compounds or the mixtures of the aluminum compounds and the chromium compounds may also be effected during the production of difluoromethane by contacting them as such with a gaseous mixture of raw materials for difluoromethane (i.e., $CH_2Cl_2$ and HF). However, it is preferred that fluorinating treatment of the aluminum compounds or the mixtures of the aluminum compounds and the chromium compounds be carried out in advance since the reaction conditions for producing difluoromethane are not necessarily identical with the conditions for the fluorinating treatment.

While the chemical composition of the catalyst obtained by the fluorinating treatment is not entirely clear, it is believed that the catalyst would be at least partly fluorinated , and an excellent catalyst having high activity and extremely minimized side reactions can be obtained. In the case of using $CrF_3$ and $AlF_3$ as a catalyst, it is unnecessary to perform the fluorinating treatment, as a matter of course.

The method for producing the catalyst (ii) comprising ferric chloride on an activated carbon carrier used in the process of the present invention is not particularly limited. For example, a determined amount of ferric chloride which is anhydrous or possesses

- 6 -

crystalline water is dissolved in a solvent, e.g., a determined amount of water or an aqueous hydrochloric acid solution, etc., in which ferric chloride is easily soluble, to prepare a solution having a determined concentration. A determined amount of activated carbon which has been previously dried is immersed in the resulting solution and allowed to reach adsorption equilibrium. Thereafter the activated carbon is removed and dried in vacuum or with heating. Alternatively, activated carbon is immersed in the aforesaid ferric chloride solution and then the dispersion was evaporated to dryness. The above catalyst can be prepared in low costs and is economically excellent.

Further when the proportion of ferric chloride to activated carbon is too small, the reaction does not proceed and the rate of conversion into $CH_2F_2$ becomes poor. It is thus preferred that the proportion of ferric chloride to be carried be large to a certain extent. The conversion rate increases as the proportion of ferric chloride increases. However, even though the quantity of ferric chloride carried increases, the conversion rate scarcely increases as the quantity of ferric chloride carried reaches to a certain level. It is preferred to choose ferric chloride generally in the range of from 0.05 to 1 part by weight, more preferably

- 7 -

in the range of from 0.1 to 0.5 part by weight, per part by weight of activated carbon.

The reaction temperature varies depending upon the proportion of HF and $CH_2Cl_2$, kind of catalyst, conditions such as contact time with catalyst, etc. However, when the temperature is too low, the reactivity becomes poor and the yield of $CH_2F_2$ becomes poor. Alternatively when the temperature is too high, there is a danger of causing problems such as deterioration of catalyst, corrosion of materials for constructing reactors, etc. In the case of using the catalyst (i), it is preferred that the reaction temperature be in the range of 200 to 500°C, and more preferably 300 to 450°C. Further in the case of using the catalyst (ii), it is preferred that the temperature be in the range of 200 to 350°C, and more preferably 250 to 320°C.

Turning to the proportion of raw materials HF and $CH_2Cl_2$, the formation of $CH_2F_2$ decreases when the proportion of HF is low. When the proportion of HF increases, the yield of $CH_2F_2$ increases. Thus, increasing the proportion of HF is effective to some extent, but, over a certain level, the yield of $CH_2F_2$ does not markedly increase even though the proportion is raised. In addition, excess or unreacted HF should be discarded or recovered. Accordingly, the increased HF proportion results in complicated treatments and economical disadvantage. It is thus preferred that the molar ratio of HF to $CH_2Cl_2$ be in the range of from 1/1 to 20/1, and more preferably is from 2/1 to 10/1.

It is necessary that the raw materials be gasified in advance using an evaporator, etc., since the reaction is a gaseous reaction. Further, the operation pressure is not particularly limited as long as it is within the range in which the raw materials and the product are not liquefied, but it is preferred from an economical viewpoint that the reaction be conducted under normal pressure or under slightly compressed pressure.

The mode of contact of the catalyst with the raw materials is not particularly limited, but fluidized bed mode or fixed bed mode, etc., may be applied. For simplicity of apparatus, the fixed bed mode is preferred.

As stated above, the process for producing $CH_2F_2$ in accordance with the present invention is an extremely excellent process from an industrial standpoint in which by-products are hardly produced and $CH_2F_2$ is obtained in high yield.

Hereafter the present invention will be described in more detail with reference with the following examples.

- 10 -

## EXAMPLE 1

In water was dissolved 300 g of $AlCl_3 \cdot 6H_2O$, and 250 g of an aqueous 46% hydrofluoric acid solution commercially available was gradually added to the resulting solution to form aluminum trifluoride. Subsequently, by maintaining the system at about 70°C under reduced pressure of about 50 mmHg, the by-produced hydrochloric acid, excess hydrofluoric acid and most of water were removed by evaporation to obtain paste-like aluminum fluoride. After molding it into a pellet of 6 mmφ × 6 mmH, the pellet was gradually heated in an $N_2$ flow to dry. After maintaining at about 400°C for 3 hours, 100g of aluminum fluoride catalyst was obtained.

This catalyst, 100 mℓ, was placed in a Hastelloy C-made reactor having an inner diameter of 20 mm and a length of 1 m. While maintaining the inner temperature of the reactor at 400°C under normal pressure, 2.1 g/hr of $CH_2Cl_2$ and 4.0 g/hr of HF were supplied into the reactor in a gaseous phase by evaporating them in an evaporator. After the system was sufficiently stabilized, the organic matter which remained after the removal of the unreacted HF and the formed HCl in the outlet gas of the reactor using an alkali was recovered for 5 hours by condensation and liquefaction.

The composition of the recovered organic matter was analyzed by gas chromatography, and as a result, the following data was obtained.

$CH_2F_2$: 4.80 g, $CH_2ClF$: 0.85 g, $CH_2Cl_2$: 1.26 g

From the results, it is understood that the $CH_2F_2$ and $CH_2ClF$ were formed in yields of 75% and 10%, respectively, based on $CH_2Cl_2$ supplied.

$CH_2ClF$ and $CH_2Cl_2$ can be used as raw materials for $CH_2F_2$ by recovering them, so that the ultimate yield of $CH_2F_2$ based on $CH_2Cl_2$ becomes higher.

EXAMPLE 2

In a Hastelloy C-made reactor having an inner diameter of 20 mm and a length of 1 m was filled up 100 ml of pellet-like activated alumina of 6 mmφ × 6 mmH.

While maintaining the inner temperature of the reactor at 350°C using a heater, $N_2$ gas was fed at a flow rate of 30 ml/min to dry. Thereafter, $N_2$ gas was discontinued and HF was slowly fed at 350°C. The feeding of HF alone was continued until the consumption of HF was not substantially observed. Then, the inner temperature of the reactor was raised to 375°C and 2.4 g/hr of $CH_2Cl_2$ and 3.9 g/hr of HF were supplied into the reactor in a gaseous phase. After the system was sufficiently stabilized, the organic matter which remained after the removal of the unreacted HF and the formed HCl in

- 12 -

the outlet gas of the reactor using an alkali was recovered for 5 hours by condensation and liquefaction.

The composition of the recovered organic matter was analyzed by gas chromatography and as a result, the following data was obtained.

$CH_2F_2$: 5.2 g, $CH_2ClF$: 1.1 g, $CH_2Cl_2$: 1.6 g

The results indicate that $CH_2F_2$ and $CH_2ClF$ were formed in yields of 71% and 11%, respectively, based on the $CH_2Cl_2$ supplied.

### EXAMPLE 3

In the same reactor was filled up 100 mℓ of the same activated alumina as used in Example 2. While maintaining the inner temperature of the reactor at 400°C using a heater, $N_2$ gas was fed at a flow rate of 30 mℓ/min to the dry activated alumina.

Subsequently, while maintaining the temperature of the reactor at about 300°C, the feeding of $N_2$ gas was discontinued. Immediately thereafter, 2.4 g/hr of $CH_2Cl_2$ and 3.9 g/hr of HF were supplied into the reactor in a gaseous phase.

As the raw material gases were fed, a sudden increase of the temperature was caused around the inlet of the reactor to exceed 400°C. The high temperature of the filled layer was successively shifted from the inlet to the central part of the layer.

During the shifting, the acidic components in the outlet gas of the reactor were captured with an alkali and the organic matter was recovered by condensation and liquefaction. The acidic components captured and the organic matter were analyzed by titration and gas chromatography, respectively. As a result, most of $CH_2Cl_2$ supplied remained unreacted, and 70% of HF was consumed. Further, a part of the catalyst after supplying the aforesaid raw material gases was taken out and its X-ray diffraction was examined. As a result, it was confirmed that the presence of $AlF_3$ was established.

This catalyst was reacted under the same conditions using the same apparatus as used in Example 2. As a result, $CH_2F_2$ and $CH_2ClF$ were formed in yields of 64% and 13%, respectively, based on the $CH_2Cl_2$ supplied.

From the foregoing, it was confirmed that the fluorinating treatment of the catalyst was possible also by gases of $CH_2Cl_2$ and HF.

### EXAMPLE 4

20 g of aluminum chloride was dissolved in 100 g of water, and 100 g of activated carbon pellet of 4 mm$\phi$ × 4 mmH which had been dried at 300°C in an $N_2$ flow was added thereto and mixed therewith. Thereafter, the mixture was dried with an evaporator

- 14 -

under reduced pressure to obtain the catalyst carried on activated carbon.. The catalyst was filled up in the same reactor as used in Example 1.

Subsequently, while flowing $N_2$ gas at a flow rate of 30 mℓ/min, drying was performed at 200°C for 2 hours and gaseous HF was fed through the reactor. The feeding of $N_2$ gas was gradually discontinued, and, at the same time, the inner temperature was heated to 400°C. After the consumption of HF was not substantially observed, 2.1 g/hr of $CH_2Cl_2$ and 4.0 g/hr of HF were supplied into the reactor in a gaseous phase while evaporating them in an evaporator. After the system was sufficiently stabilized, the amount of the organic matter recovered in a manner similar to Example 1 was determined to obtain $CH_2F_2$: 4.5 g, $CH_2ClF$: 0.93 g, $CH_2Cl_2$: 1.4 g. The results indicate that $CH_2F_2$ and $CH_2ClF$ were formed in yields of 70% and 11%, respectively, based on the $CH_2Cl_2$ supplied.

EXAMPLE 5

After thoroughly mixing 30 g of commercially available $AlF_3$ and 100 g of powdery activated carbon, the mixture was molded into a pellet of 6 mmφ × 6 mmH.

100 mℓ of this catalyst was placed in the same reactor as used in Example 1, and the system was maintained at 400°C for 3 hours in an $N_2$ flow.

- 14 -

Subsequently, while maintaining the inner temperature of the reactor at 390°C, 2.10 g/hr of $CH_2Cl_2$ and 4.0 g/hr of HF were supplied into the reactor in a gaseous phase while evaporating them in an evaporator. After the system was sufficiently stabilized, the organic matter which remained after the removal of the unreacted HF and the formed HCl in the outlet gas of the reactor using an alkali was recovered for 5 hours by condensation and liquefaction.

The composition of the recovered organic matter was analyzed by gas chromatography and as a result, the following data was obtained.

$CH_2F_2$: 4.6 g, $CH_2ClF$: 1.0 g, $CH_2Cl_2$: 1.40 g

The results indicate that $CH_2F_2$ and $CH_2ClF$ were formed in yields of 72% and 12%, respectively, based on the $CH_2Cl_2$ supplied.

### EXAMPLE 6

360 g of $AlCl_3 \cdot 6H_2O$ was dissolved in water and 250 g of HF was gradually added to the resulting solution to react them. Thereafter, the system was heated in an $N_2$ flow, the moisture, formed HCl and excess HF were evaporated off and removed to obtain $AlF_3$. Commercially available $CrF_3 \cdot 3H_2O$ powder, 63 g, was thoroughly mixed with the whole amount of this $AlF_3$ to obtain a mixture of $CrF_3$ and $AlF_3$. The mixture was molded into a pellet

of 6 mmφ × 6 mmH. Thereafter, the pellet was dried by heating at about 350°C for 3 hours in an $N_2$ flow to obtain a pellet-like mixture of $AlF_3$ and $CrF_3$ having a ratio of Al weight/Cr weight in the compound of 2/1.

This catalyst, 100 ml, was filled up in a Hastelloy C-made reactor having an inner diameter of 20 mm and a length of 1 m. While maintaining the inner temperature of the reactor at 330°C under normal pressure, 6.3 g/hr of $CH_2Cl_2$ and 7.4 g/hr of HF were supplied into the reactor in a gaseous phase by evaporating them in an evaporator. After the system was sufficiently stabilized, the organic matter which remained after the removal of the unreacted HF and the formed HCl in the outlet gas of the reactor using an alkali was recovered for 5 hours by condensation and liquefaction.

The composition of the recovered organic matter was analyzed by gas chromatography and as a result, the following data was obtained.

$CH_2F_2$: 12.7 g, $CH_2ClF$: 3.8 g, $CH_2Cl_2$: 6.0 g

The results indicate that $CH_2F_2$ and $CH_2ClF$ were formed in yields of 66% and 15%, respectively, based on the $CH_2Cl_2$ supplied, and by-products were scarcely produced.

- 16 -

EXAMPLE 7

In the same reactor as used in Example 6 there was placed 100 mℓ of a pellet molded into 4 mmφ × 4 mmH, composed of 40 wt% of chromic trioxide (chromia) and 60 wt% of aluminum trioxide (alumina).

Then, while flowing $N_2$ gas at a flow rate of 30 mℓ/min, the inner temperature of the reactor was gradually raised using a heater, and drying was performed at 200°C. Thereafter HF gas was added thereto, the feeding of $N_2$ gas was slowly discontinued, and, at the same time, the inner temperature was raised to 350°C. After the consumption of HF was not observed, the feeding of HF was discontinued to obtain the fluorinated catalyst having a weight ratio of Aℓ/Cr of 1.2/1. Subsequently, while maintaining the inner temperature of the reactor at 300°C, 6.2 g/hr of $CH_2Cℓ_2$ and 6.6 g/hr of HF were supplied into the reactor in a gaseous phase by evaporating them in an evaporator. After the system was sufficiently stabilized, the organic matter which remained after the removal of the unreacted HF and the formed HCl in the oulet gas of the reactor using an alkali was recovered for 5 hours by condensation and liquefaction.

The composition of the recovered organic matter was analyzed by gas chromatography, and, as a result, the following data was obtained.

- 17 -

$CH_2F_2$: 11.9 g, $CH_2C\ell F$: 3.5 g, $CH_2C\ell_2$: 7.1 g

The results indicate that $CH_2F_2$ and $CH_2ClF$ were formed in yields of 63% and 14%, respectively, based on the $CH_2C\ell_2$ supplied.

## EXAMPLE 8

26 g of $CrC\ell_3 \cdot 6H_2O$ and 45 g of $A\ell C\ell_3 \cdot 6H_2O$ were dissolved in water to prepare an aqueous solution. After 100 g of an activated carbon pellet 4 mm$\phi$ × 4 mmH which had been dried at 300°C for 2 hours in an $N_2$ flow was mixed with the aqueous solution, the mixture was dried in an evaporator in vacuum to deposit about 0.15 part by weight of $CrC\ell_3$ and 0.25 part by weight of $A\ell C\ell_3$ per 1 part by weight of activated carbon.

100 m$\ell$ of the aforesaid activated carbon having deposited thereon $A\ell C\ell_3$ and $CrC\ell_3$ was placed in a Hastelloy C-made reactor having an inner diameter of 20 mm and a length of 1 m, and while feeding $N_2$ gas at a flow rate of 30 m$\ell$/min, the inner temperature of the reactor was slowly raised using a heater and dried at 200°C. Then, HF gas was added thereto, the flow of $N_2$ gas was slowly discontinued, and the inner temperature was raised to 350°C. After consumption of HF passed through the reactor was not observed, the feeding of HF was discontinued, and thus

- 18 -

a catalyst was obtained.

Next, while maintaining the inner temperature of the reactor at 300°C, 7.6 g/hr of $CH_2Cl_2$ and 7.1 g/hr of HF were supplied into the reactor in a gaseous phase while evaporating them in an evaporator. After the system was sufficiently stabilized, the organic matter which remained after the removal of the unreacted HF and the formed HCl in the outlet gas of the reactor using an alkali was recovered for 5 hours by condensation and liquefaction.

The composition of the recovered organic matter was analyzed by gas chromatography and as a result, the following data was obtained.

$CH_2F_2$: 14.1 g, $CH_2ClF$: 4.6 g, $CH_2Cl_2$: 9.1 g

The results indicate that $CH_2F_2$ and $CH_2ClF$ were formed in yields of 60.6% and 15.0%, respectively, based on the $CH_2Cl_2$ supplied.

Further, the total number of moles of the $CH_2F_2$ and $CH_2ClF$ formed and the unreacted $CH_2Cl_2$ exceeded 99% of the number of moles of the $CH_2Cl_2$ supplied, indicating that by-products were scarcely produced.

## EXAMPLE 9

After thoroughly mixing 50 g of $AlF_3$ powders and 20 g of $CrF_3 \cdot 3H_2O$ powders, commercially available, and 100 g of powdery activated carbon , the mixture was molded into a pellet of about 6 mmφ × 6 mmH, whereby a catalyst composed of $AlF_3$ and $CrF_3$ in the proportion of about 0.4 part by weight of chromium per 1 part by weight of aluminum was obtained.

100 mℓ of this catalyst was placed in a Hastelloy C-made reactor having an inner diameter of 20 mm and a length of 1 m, and was dried by heating at about 350°C for 2 hours in an $N_2$ flow.

The feeding of $N_2$ was discontinued. Subsequently, while maintaining the inner temperature of the reactor at 300°C, 7.3 g/hr of $CH_2Cl_2$ and 10.3 g/hr of HF were supplied into the reactor by evaporating them in an evaporator. After the system was sufficiently stabilized, the organic matter which remained after the removal of the unreacted HF and the formed HCl in the outlet gas of the reactor using an alkali was recovered for 5 hours by condensation and liquefaction.

The composition of the recovered organic matter was analyzed by gas chromatography, and, as a result, the following data was obtained.

- 20 -

$CH_2F_2$: 14.6 g, $CH_2ClF$: 4.1 g, $CH_2Cl_2$: 7.7 g

The results indicate that $CH_2F_2$ and $CH_2ClF$ were formed in yields of 65% and 14%, respectively, based on the $CH_2Cl_2$ supplied.

EXAMPLE 10

In about 200 g of water was dissolved 30 g of anhydrous ferric chloride to prepare an aqueous solution of ferric chloride. To the aqueous solution was added 100 g of activated carbon of a cylindrical shape having 4 mmφ × 4 mmH, previously dried at about 300°C in an $N_2$ flow. After stirring the mixture, water was evaporated off by heating to prepare a catalyst.

This catalyst, 100 mℓ, was filled up in a reactor having a diameter of 20 mm and further thoroughly dried by heating at about 300°C in an $N_2$ flow. Then, while maintaining the inner temperature of the reactor at 300°C in an $N_2$ flow under normal pressure, 2.46 g/hr of $CH_2Cl_2$ and 5.22 g/hr of HF were supplied into the reactor in a gaseous phase by evaporating them in an evaporator.

After the system was sufficiently stabilized, the organic matter which remained after the removal of the unreacted HF and the formed HCl in the outlet gas of the reactor using an alkali was recovered for 5 hours by condensation and liquefaction.

The composition of the recovered organic matter was analyzed by gas chromatography and as a result, the following data was obtained.

$CH_2F_2$: 5.73 g, $CH_2ClF$: 0.89 g, $CH_2Cl_2$: 0.98 g

The results indicate that $CH_2F_2$ and $CH_2ClF$ were formed in yields of 76% and 9%, respectively, based on the $CH_2Cl_2$ supplied. Thus the $CH_2F_2$ was obtained in an extremely high yield and the production of the by-products were extremely minimized.

As is evident from the above examples, the process of the present invention is an excellent process for producing $CH_2F_2$ in which the formation yields of $CH_2F_2$ is very high based on $CH_2Cl_2$, undesired side-reaction is hardly noted, and the by-produced $CH_2ClF$ and unreacted $CH_2Cl_2$ can be recovered and used as raw materials for $CH_2F_2$ so that the yield becomes higher.

While the invention has been described in detail and with reference to specific embodiments thereof, it will be apparent to one skilled in the art that various changes and modifications can be made therein without departing from the spirit and scope thereof.

- 22 -

23.

WHAT IS CLAIMED IS:

1. A process for producing difluoromethane which comprises reacting dichloromethane and hydrogen fluoride in a gaseous phase in the presence of at least one catalyst selected from (i) a catalyst consisting essentially of aluminum fluoride or a mixture of aluminum fluoride and chromium fluoride and (ii) a catalyst comprising from 0.05 to 1 part by weight of ferric chloride on 1 part by weight of activated carbon carrier.

2. A process for producing difluoromethane as in Claim 1, wherein said catalyst is aluminum fluoride, a catalyst obtained by mixing aluminum fluoride with a carrier followed by molding or a catalyst obtained by depositing aluminum fluoride on a carrier.

3. A process for producing difluoromethane as in Claim 1, wherein said catalyst is a mixture of aluminum fluoride and chromium fluoride, a catalyst obtained by mixing said mixture with a carrier followed by molding, or a catalyst obtained by depositing said mixture on a carrier.

4. A process for producing difluoromethane as in Claim 1, wherein said catalyst is a catalyst obtained by depositing from 0.05 to 1 part by weight of ferric chloride on 1 part by weight of activated carbon carrier.

5. A process for producing difluoromethane as in Claim 2, wherein said gaseous reaction is performed at 200 to 500°C.

6. A process for producing difluoromethane as in Claim 3, wherein said gaseous reaction is performed at 200 to 500°C.

7. A process for producing difluoromethane as in Claim 4, wherein said gaseous reaction is performed at 200 to 350°C.

8. A process for producing difluoromethane as in Claim 5, wherein said aluminum fluoride is obtained by subjecting an aluminum compound to a fluorinating treatment.

9. A process for producing difluoromethane as in Claim 6, wherein said mixture of aluminum fluoride and chromium fluoride is obtained by subjecting an aluminum compound and a chromium compound to a fluorinating treatment.

10. A process for producing difluoromethane as in Claim 6, wherein the chromium fluoride is chromium trifluoride.

11. A process for producing difluoromethane as in Claim 8, wherein said aluminum compound is selected from the group consisting of an oxide, chloride, hydroxide and nitrate of aluminum.

12. A process for producing difluoromethane as in Claim 9, wherein said aluminum compound is selected from the group consisting of an oxide, chloride, hydroxide and nitrate of aluminum and said chromium compound is selected from the group consisting of chromium oxide, chromium hydroxide, chromium chloride, chromium nitrate, and a chromate.

13. A process for producing difluoromethane as in Claim 9, wherein a weight mixing ratio of Aℓ in said aluminum compound to Cr in said chromium compound is 0.3/1 to 20/1.

14. A process for producing difluoromethane as in Claim 2, wherein said carrier is activated carbon.

15. A process for producing difluoromethane as in Claim 3, wherein said carrier is activated carbon.

16. A process for producing difluoromethane as in claim 6, wherein said catalyst has a Al/Cr weight ratio of from 0.3/1 to 20/1.